# EUROPEAN PATENT APPLICATION

(11) **EP 4 454 596 A1**
(43) Date of publication of application: **30.10.2024**
(21) Application number: 22911245.3
(22) Date of filing: 20.12.2022
(51) Int. Cl.: A61B 90/14

(54) **HEAD PIN**

(30) Priority: 20.12.2021 JP 2021206525
(71) Applicant: Spine-Tec Inc, Tokyo 105-0003 (JP); Takai Corporation, Mino-Shi, Gifu 501-3712 (JP)
(72) Inventor: KOSEKI, Hirokazu, Tokyo 105-8461 (JP); MORI, Ryosuke, Tokyo 105-8461 (JP); MORITA, Kohei, Tokyo 105-8461 (JP); MURAYAMA, Yuichi, Tokyo 105-8461 (JP); TSUCHIDA, Kenji, Mino-shi, Gifu 501-3712 (JP); FURUTA, Yasuhiro, Mino-shi, Gifu 501-3712 (JP)
(74) Representative: SSM Sandmair
(86) International application number: PCT/JP2022/046974
(87) International publication number: WO 2023/120543

(57) **Abstract**

The invention provides a skull pin with excellent disposability and the ability to reduce artifacts. The skull pin of the present invention is capable of fixing the head of an animal and comprises a body part of the skull pin having a first reinforced plastic, which includes 30 to 70 wt% of a first reinforcing fiber and 30 to 70 wt% of a first resin, and a tip part fixed to the tip of the body part, which is capable of contacting the head and includes at least one of metal, ceramics, cermet, and a second reinforced plastic.

## Description

### TECHNICAL FIELD

This invention relates to a skull pin that can fix the head of an animal.

### BACKGROUND OF INVENTION

In order to fix the head of an animal (especially, a human) in surgery in the field of neurosurgery and the like, a skull pin including a tip that pierces partway into the head (skull) has been developed (see Patent Document 1).

Conventionally, in order to maintain the strength to fix the head, a skull pin whose tip is sapphire has been proposed, which is made entirely of metal (such as stainless steel). In addition, in order to maintain the hygiene of the tip of the skull pin that invades the living body, a skull pin with a cap made of metal or other material attached to and removed from the tip has been proposed.

### CITATION LIST

### PATENT LITERATURE

Patent Document 1: JP2005-342335A

### SUMMARY

### [Problem to be solved]

Skull pins made entirely of metal (such as stainless steel) cause artifacts when medical images of the head are captured, and this hinders the surgeon from performing surgery while checking the medical images. Sapphire skull pins are not suitable as disposable skull pins because they do not reduce artifacts and are expensive. In addition, a cap made of metal or other material that can be attached to and removed from the tip is superior in disposability, but it is necessary to cover the invasive area extensively, so it cannot reduce the artifacts caused by the cap made of metal or other material.

The skull pin of the present invention is a skull pin capable of fixing the head of an animal, the skull pin comprising: a body member of a first reinforced plastic including 30 wt% to 70 wt% of first reinforcing fibers and 30 wt% to 70 wt% of a first resin; and a tip member configured to be fixed to a tip of the body member, to contact the head, and to include at least one of metal, ceramics, cermet and a second reinforced plastic.

The present invention provides a skull pin that has excellent disposability and reduces artifacts.

### BRIEF DESCRIPTION OF THE DRAWINGS

Figure 1 is side and front views of the skull pin in the embodiment.
Figure 2 is a perspective view of the skull pin in the embodiment.
Figure 3 is a table showing the experimental results of compression strength and the presence or absence of internal cavities when varying the composition ratio (by weight) of reinforcing fibers and resin in the body member.
Figure 4 is a diagram illustrating the formation of internal cavities.
Figure 5 is a diagram showing examples of the shape and size of the tip.
Figure 6 is a diagram showing the experimental results of piercing a simulated bone with the skull pin.
Figure 7 is a diagram illustrating the artifacts of a conventional skull pin.
Figure 8 is a diagram illustrating the artifacts of the skull pin in the embodiment.
Figure 9 is a table evaluating the artifacts of the skull pin.
Figure 10 is side, front, and perspective views of other skull pins in the embodiment.

### DESCRIPTION OF EMBODIMENTS

Firstly, the inventors have discovered the appropriate composition ratio of reinforcing fibers and resin in a skull pin capable of fixing an animal's head, achieving both suitable compressive strength and low artifacts. Secondly, for ensuring reliable fixation of the head, the inventors have identified at least one of the material, shape, and size for the tip member of the skull pin, which requires high strength, to achieve both reliability in head fixation and low artifacts by using a high-strength material for the tip member.

According to this embodiment, a skull pin capable of achieving low artifacts can be provided. Furthermore, because the skull pin of this embodiment mainly comprises reinforcing fibers and resin as its principal components, it can be easily mass-produced through injection molding using molds, thereby providing an affordable and disposable skull pin with excellent disposability.

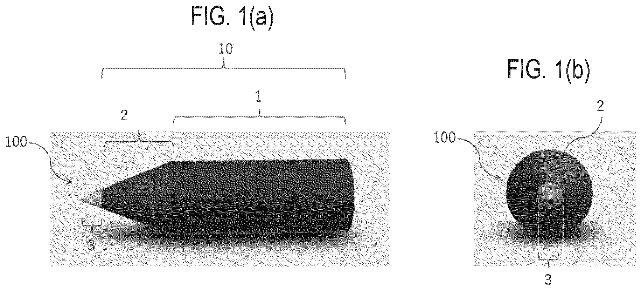

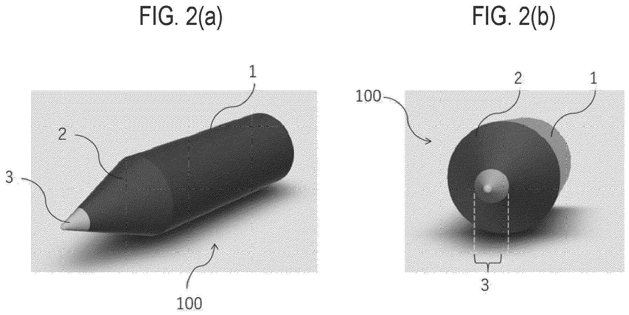

The skull pin of this embodiment will be described using drawings. Figure 1 is a side and front view of the skull pin in this embodiment. Figure 2 is a perspective view of the skull pin in this embodiment.

As shown in Figures 1 and 2, the skull pin 100 comprises a body member 10 (including a shaft member 1 and a tapered member 2) and a tip member 3. The tip member 3 is fixed to the tip of the body member 10.

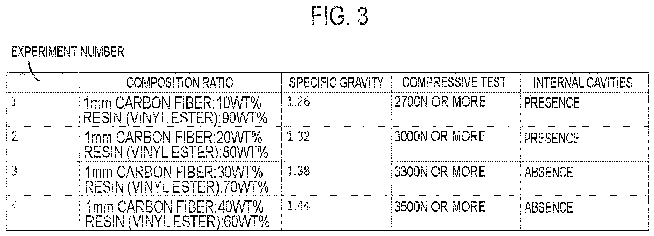

Figure 3 shows a table of the experimental results for the compressive strength and the presence or absence of internal cavities when the composition ratio (weight %: wt%) of reinforcing fibers (1mm carbon fibers) and resin (vinyl ester) in the body member 10 is varied. Since the body member 10 is primarily composed of reinforcing fibers and resin, achieving low artifacts is possible, but appropriate compressive strength is required.

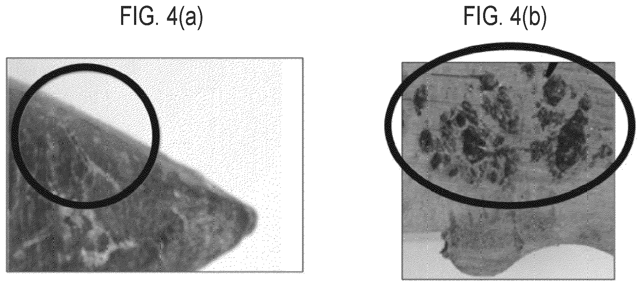

The experimental results showed that as the composition ratio of reinforcing fibers increases, the compressive strength also increases. This indicates that the compressive strength depends on the composition ratio of reinforcing fibers. Moreover, as shown in Figure 4A, it was found that increasing the composition ratio of resin leads to the formation of internal cavities, indicating that the presence of internal cavities depends on the resin composition ratio. Indeed, as shown in Figure 4B, internal cavities were confirmed in skull pins formed solely of resin.

In experiment number 1 and 2, while sufficient compressive strengths of 2700N and 3000N, respectively, were observed, the performance of the skull pin cannot be guaranteed due to internal cavities caused by a high resin composition ratio. That is, depending on the location and shape of the internal cavities, the skull pin might not achieve the required compressive strength. Therefore, the experimental results suggest that the resin composition ratio may be below 70 wt%.

In experiment number 3 and 4, sufficient compressive strengths of 2700N and 3000N, respectively, were observed without the occurrence of internal cavities, indicating that to achieve the compressive strength for the skull pin, the body member 10 needs a composition ratio of over 30 wt% of reinforcing fibers (first reinforcing fibers).

On the other hand, setting the composition ratio of the first reinforcing fibers above 30 wt% and the resin composition ratio below 70 wt% can increase the compressive strength while preventing internal cavities in the body member 10. However, when the resin composition ratio was less than 30 wt%, the viscosity during injection molding of the body member 10 was insufficient, leading to unsuccessful injection molding.

Therefore, to achieve sufficient compressive strength for the body member 10 and suitability for injection molding, the composition ratio for the body member 10 may be 30 wt% to 70 wt% of the first reinforcing fibers and 30 wt% to 70 wt% of resin. Since reinforcing fibers are more expensive than resin, to provide a more affordable and disposable skull pin, it is preferable that the composition ratio for the body member 10 is 30 wt% to 50 wt% of the first reinforcing fibers and 50 wt% to 70 wt% of resin.

Thus, the body member 10 has a first reinforced plastic containing 30 wt% to 70 wt% of the first reinforcing fibers and 30 wt% to 70 wt% of the first resin. The first reinforced plastic preferably is carbon fiber reinforced plastic, but it could also be glass fiber reinforced plastic, carbon fiber reinforced plastic, boron fiber reinforced plastic, aramid fiber reinforced plastic, Kevlar fiber reinforced plastic, Dyneema fiber reinforced plastic, or Zylon fiber reinforced plastic, any of which can achieve sufficient compressive strength for the body member 10. Additionally, because the occurrence of internal cavities and viscosity during injection molding depend on the resin composition ratio, the composition ratio for the body member 10 is similar for these reinforcing fibers.

The tip member 3 is capable of contacting the head of an animal and, to provide a skull pin that securely fixes the head, it is preferably capable of piercing the animal's head. Due to the particular forces applied to the tip member 3, a higher compressive strength than that of the body member 10 is required. In this case, the higher compressive strength compared to the body member 10 depends on at least one of the material, shape, and size of the tip member 3.

Furthermore, artifacts caused by the tip member 3 also depend on at least one of the material, shape, and size of the tip member 3. For instance, if the material of the tip member 3 is reinforcing fibers, low artifacts can be achieved regardless of the shape and size. If the material of the tip member 3 is metal, it tends to produce artifacts, but selecting the appropriate shape and size can achieve low artifacts.

First, the material of the tip member 3 includes at least one of metal, ceramics, cermet, and a second reinforced plastic.

To ensure reliable fixation of the head, the material of the tip member 3 is preferably at least one of metal, ceramics, and cermet. Moreover, to reduce artifacts, the material of the tip member 3 is preferably reinforced plastic.

Next, the shape and size of the tip member 3 include at least one shape of a substantially conical, frustoconical, and rotational body, and to reduce artifacts, it is preferable that the size has a maximum length of the base between 1.5mm to 3mm.

Note that the size of the tip member 3 is set based on metal, which tends to cause artifacts, and with other materials, artifacts are further reduced.

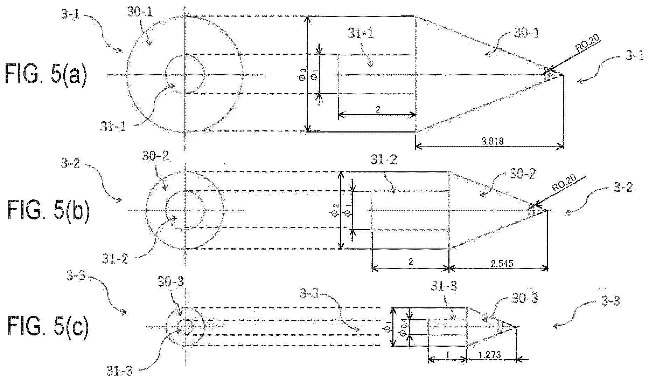

Figure 5 is a diagram showing examples of the shape and size of the tip member. The material is metal (titanium). In Figure 5, the tip members 3-1, 3-2, 3-3 include substantially conical (rotational bodies) 30-1, 30-2, 30-3, with the base length of each substantially conical (rotational body) being 1mm, 2mm, and 3mm, respectively.

Furthermore, the tip members 3-1, 3-2, 3-3 include protrusions 31-1, 31-2, 31-3 extending towards the body member 10 side, and these protrusions allow the tip members 30-1, 30-2, 30-3 to be fixed to the body member 10 by being embedded in it.

The protrusions 31-1, 31-2, 31-3 in Figure 5 are cylindrical, but to enhance the fixing strength to the body member 10, the protrusion 31 may include at least one of a tapered shape, screw shape, flange shape, and arrowhead shape. To further enhance the fixing strength to the body member 10, it is preferable for the protrusion 31 to be a flange shape. Also, to provide a more affordable and disposable skull pin, it is preferable for the protrusion 31 to be a screw shape, allowing it to be detachable from the body member 10. In this case, the embedding hole in the body member 10 (not shown) has a shape that screws onto the screw-shaped protrusion 31.

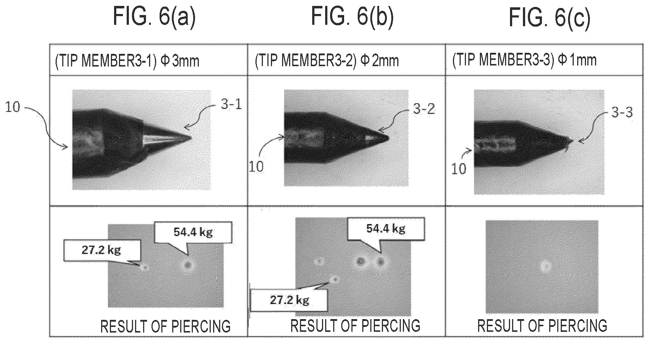

Figure 6 is a diagram showing the experimental results of piercing a simulated bone with skull pins equipped with tip members 30-1, 30-2, 30-3 (made of titanium), respectively. The protrusions of each tip member 30-1, 30-2, 30-3 (not shown) extend towards the body member 10 side and are embedded in it. In Figure 6, the results are shown where the skull pin is pierced partway into the simulated bone with forces of 27.2kg (60 pounds) and 54.4kg (120 pounds) applied to the tip member 3.

Figure 6A is a diagram showing the experimental results of piercing a simulated bone with a skull pin equipped with tip member 30-1. In both weights applied, tip member 30-1 pierced partway into the simulated bone and showed sufficient compressive strength and fixing strength.

Figure 6B is a diagram showing the experimental results of piercing a simulated bone with a skull pin equipped with tip member 30-2. In both weights applied, tip member 30-2 pierced partway into the simulated bone and showed sufficient compressive strength and fixing strength.

Figure 6C is a diagram showing the experimental results of piercing a simulated bone with a skull pin equipped with tip member 30-3. Before reaching the weight of 27.2kg (60 pounds), the tip of tip member 30-2 bent, and it could not show sufficient compressive strength and fixing strength.

Note that, although not shown in Figure 6, similar experiments were conducted with a tip member 3 that includes a substantially conical (rotational body) with a base length of 1.5mm, and in both weights applied, tip member 30-2 pierced partway into the simulated bone and showed sufficient compressive strength and fixing strength.

Therefore, based on the experimental results, to achieve sufficient compressive strength and fixing strength for the tip member 3, the base length of the substantially conical (rotational body) included in the tip member 3 may be at least 1.5mm. To consider low artifacts, the base length of the substantially conical (rotational body) included in the tip member 3 may be no more than 3mm. Considering both sufficient compressive strength and fixing strength, as well as low artifacts for the tip member 3, the base length of the substantially conical (rotational body) included in the tip member 3 is preferably between 1.5mm and 2.5mm, and more preferably between 1.5mm and 2mm.

Next, the results of the artifact evaluation experiments with skull pins having base lengths of the substantially conical (rotational body) included in the tip member 3 of 1mm, 1.5mm, 2mm, and 3mm are shown.

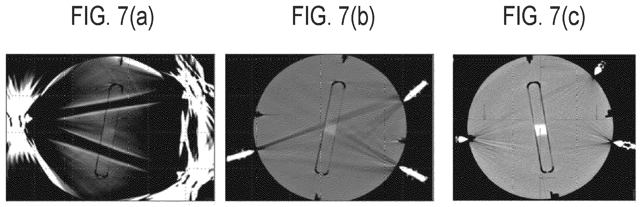

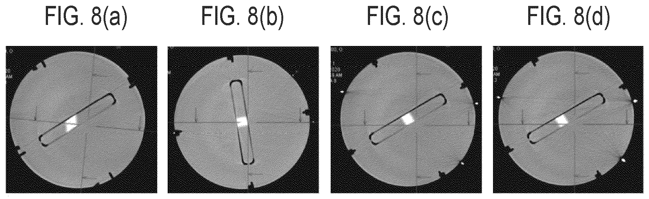

Figure 7 is a diagram showing the artifacts of a conventional skull pin. Figure 8 is a diagram showing the artifacts of the skull pin in this embodiment. Each skull pin was fixed at three locations on a phantom mimicking an animal's head, and CT images (cone-beam CT images) were taken.

Figure 7A shows the artifacts of a stainless steel skull pin. Figure 7B shows the artifacts of a sapphire skull pin. Figure 7C shows the artifacts of a skull pin with a stainless steel cap installed on the tip member.

Figure 8A shows the artifacts caused by a skull pin with a base length of the substantially conical (rotational body) included in the tip member 3 of 1mm. Figure 8B shows the artifacts caused by a skull pin with a base length of 1.5mm. Figure 8C shows the artifacts caused by a skull pin with a base length of 2mm. Figure 8D shows the artifacts caused by a skull pin with a base length of 3mm.

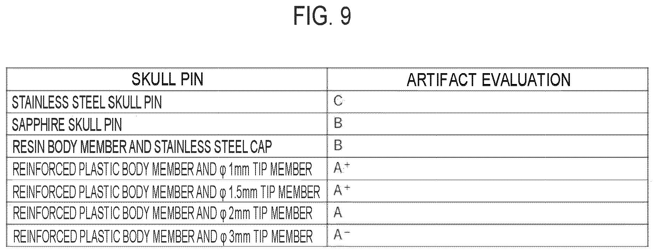

Figure 9 shows a table evaluating the artifacts of each skull pin. In the CT (Computed Tomography) images of Figure 7, artifacts radiated from the tip member of the skull pin, and the evaluation was low, C and B. In the CT images of Figure 8, as the base length of the substantially conical (rotational body) included in the tip member 3 increased, artifacts radiated from the tip member of the skull pin, but the evaluation was high, A+, A, and A-. The evaluation is acceptable up to A-.

Therefore, considering the sufficient compressive strength and fixing strength of the tip member 3, the base length of the substantially conical (rotational body) included in the tip member 3 may be at least 1.5mm. Considering low artifacts, the base length of the substantially conical (rotational body) included in the tip member 3 may be no more than 3mm. Considering both sufficient compressive strength and fixing strength, as well as low artifacts for the tip member 3, the base length of the substantially conical (rotational body) included in the tip member 3 is preferably between 1.5mm and 2.5mm, and more preferably between 1.5mm and 2mm.

In addition to titanium, the tip member 3 may include at least one metal such as stainless steel, iron, nickel, cobalt, and cemented carbide.

While the embodiments pertaining to this invention have been described, the invention is not limited to these and can be modified or transformed within the scope described in the claims.

For similar reasons to the first reinforced plastic, the second reinforced plastic may be a reinforced plastic containing 30 wt% to 70 wt% of the second reinforcing fibers and 30 wt% to 70 wt% of the second resin. The second reinforced plastic is preferably a reinforced plastic with a higher compressive strength than the first reinforcing fibers. Therefore, the second reinforced plastic is preferably a reinforced plastic containing 50 wt% to 70 wt% of the second reinforcing fibers and 30 wt% to 50 wt% of the second resin. Like the first reinforced plastic, the second reinforced plastic is preferably carbon fiber reinforced plastic, but other types of reinforced plastics are also acceptable.

Furthermore, the resin includes at least one of thermosetting resin and thermoplastic resin. For example, the resin can be selected from epoxy, phenolic, unsaturated polyester resins, and so on.

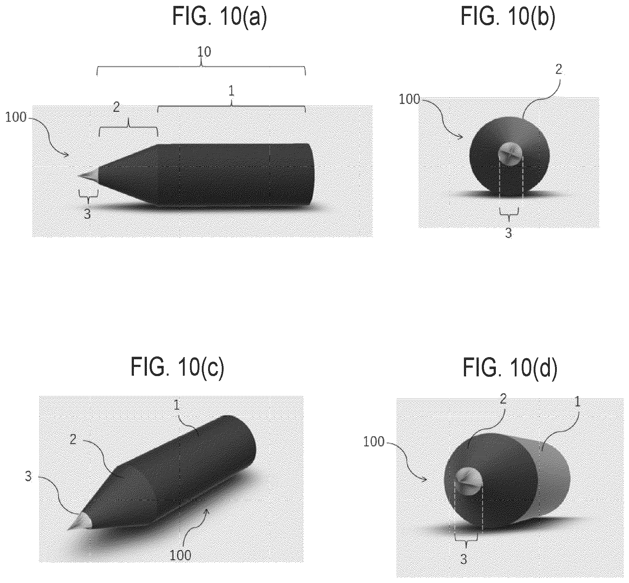

Moreover, the tip member 3 may have a shape as shown in Figure 10. This shape is also included in the substantially conical category.

### [Industrial Applicability]

This invention is useful as a skull pin with excellent disposability and capable of reducing artifacts.

### [Description of Reference Numerals]

- 1: ... Shaft member
- 2: ... Tapered member
- 3, 20: ... Tip member
- 10: ... Body member
- 31: ... Protrusion
- 100: ... Skull pin

## Claims

1. A skull pin capable of fixing the head of an animal, the skull pin comprising:
a body member of a first reinforced plastic including 30 wt% to 70 wt% of a first reinforcing fiber and 30 wt% to 70 wt% of a first resin; and
a tip member configured to be fixed to a tip of the body member, to contact the head, and to include at least one of metal, ceramics, cermet and a second reinforced plastic.

2. The skull pin according to claim 1, **characterized in that** the second reinforced plastic includes a reinforced plastic containing 30 wt% to 70 wt% of a second reinforcing fiber and 30 wt% to 70 wt% of a second resin.

3. The skull pin according to claim 1 or claim 2, **characterized in that** the reinforced plastic is at least one of carbon fiber reinforced plastic, glass fiber reinforced plastic, carbon fiber reinforced plastic, boron fiber reinforced plastic, aramid fiber reinforced plastic, Kevlar fiber reinforced plastic, Dyneema fiber reinforced plastic, and Zylon fiber reinforced plastic.

4. The skull pin according to any one of claims 1 to 3, **characterized in that** the resin includes at least one of a thermosetting resin and a thermoplastic resin.

5. The skull pin according to any one of claims 1 to 4, **characterized in that** the reinforcing fiber is a carbon fiber with a length of 0.5 mm to 5 mm.

6. The skull pin according to any one of claims 1 to 5, wherein the tip member includes at least one of stainless steel, titanium, iron, nickel, cobalt, and cemented carbide as the metal, and includes at least one shape of conical, truncated cone, and rotational body, with the maximum length of the bottom surface of the shape being 1.5 mm to 3 mm.

7. The skull pin according to any one of claims 1 to 6, **characterized in that** the tip member includes at least one of zirconia, alumina, silicon carbide, silicon nitride, sialon, cordierite, ferrite, barium titanate, lead zirconate titanate, forsterite, zircon, mullite, steatite, and aluminum nitride as the ceramics, and includes at least one shape of conical and truncated cone, with the maximum length of the bottom surface of at least one of the conical and the truncated cone being 1.5 mm to 3 mm.

8. The skull pin according to any one of claims 1 to 7, **characterized in that** the tip member includes at least one of carbon fiber reinforced plastic, glass fiber reinforced plastic, carbon fiber reinforced plastic, boron fiber reinforced plastic, aramid fiber reinforced plastic, Kevlar fiber reinforced plastic, Dyneema fiber reinforced plastic, and Zylon fiber reinforced plastic as the second reinforced plastic, and includes at least one shape of conical, truncated cone, and rotational body, with the maximum length of the bottom surface of at least one of the conical, the truncated cone, and the rotational body being 1.5 mm to 3 mm.

9. The skull pin according to any one of claims 1 to 8, wherein the tip member includes a protrusion extending towards the body part, and the protrusion, by being embedded in the body member, enables the fixation of the tip member to the body member.

10. The skull pin according to any one of claims 1 to 9, **characterized in that** the protrusion includes at least one of a cylindrical shape, tapered shape, screw shape, flange shape, and arrowhead shape.
